# EUROPÄISCHE PATENTSCHRIFT

(11) **EP 3 640 231 B1**
(45) Veröffentlichungstag und Bekanntmachung des Hinweises auf die Patenterteilung: **14.07.2021**
(21) Anmeldenummer: 18201329.2
(22) Anmeldetag: 18.10.2018
(51) Int. Cl.: C07C 5/327, C07C 4/04, C07C 7/00, C07C 7/04, C07C 11/04

(54) **VERFAHREN ZUR GEWINNUNG VON ETHYLEN**
METHOD FOR RECOVERING ETHYLENE
PROCÉDÉ DESTINÉ À L'OBTENTION D'ÉTHYLÈNE

(43) Veröffentlichungstag der Anmeldung: 22.04.2020
(73) Patentinhaber: Linde GmbH, 82049 Pullach (DE)
(72) Erfinder: HÖFEL, Torben, 81543 München (DE)

(56) Entgegenhaltungen:
- Heinz Zimmermann ET AL: "Ethylene" In: "Ullmann's Encyclopedia of Industrial Chemistry", 15. April 2009 (2009-04-15), Wiley-VCH Verlag, Weinheim, XP055007506, ISBN: 978-3-52-730673-2 DOI: 10.1002/14356007.a10_045.pub3, * Abschnitt 5.3. *

## Beschreibung

Die vorliegende Erfindung betrifft ein Verfahren zur Gewinnung von Ethylen.

### Stand der Technik

Olefine wie Ethylen oder Propylen, aber auch Diolefine wie Butadien sowie Aromaten können durch Dampfspalten (engl. Steam Cracking) aus Paraffinen hergestellt werden. Entsprechende Verfahren sind seit langem bekannt. Zu Details sei auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Beim Dampfspalten wird ein sogenanntes Spaltgas (engl. Cracked Gas) erhalten, das neben den Zielprodukten nicht umgesetzte Kohlenwasserstoffe und unerwünschte Nebenprodukte enthält. Dieses Spaltgas wird in bekannten Verfahren zunächst einer Aufbereitung unterworfen, bevor es einer Fraktionierung bzw. Trennung zur Gewinnung unterschiedlicher Kohlenwasserstoffe bzw. Fraktionen zugeführt wird. Details sind in dem zitierten Artikel insbesondere in Abschnitt 5.3.2.1, "Front-End Section" und 5.3.2.2., "Hydrocarbon Fractionation Section", beschrieben.

Eine entsprechende Aufbereitung umfasst insbesondere eine sogenannte Sauergasentfernung, bei der Komponenten wie Kohlendioxid, Schwefelwasserstoff und Mercaptane aus dem Spaltgas abgetrennt werden. Das Spaltgas wird typischerweise vor und nach einer entsprechenden Behandlung verdichtet. Beispielsweise kann das Spaltgas einem sogenannten Rohgasverdichter auf einem Zwischendruckniveau entnommen, der Sauergasentfernung unterworfen, und anschließend in dem Rohgasverdichter weiter verdichtet werden.

Mitunter ist es erwünscht, durch Dampfspalten ethanreiche Einsätze unter Bildung möglichst geringer Mengen an Nebenprodukten zu Ethylen umzusetzen. In diesem Zusammenhang werden neben nicht umgesetztem Ethan auch beim Dampfspalten gebildete Kohlenwasserstoffe drei und vier Kohlenstoffatomen typischerweise wieder in den oder die verwendeten Reaktoren zurückgeführt. Um eine einzelne Hydrierung dieser rückgeführten Fraktionen zu vermeiden, kann eine Hydrierung des gesamten Spaltgases im Zuge der Aufbereitung (sogenannte Rohgashydrierung) erfolgen.

Da die zurückgeführten Kohlenwasserstoffe mit drei und vier Kohlenstoffatomen typischerweise einen geringen Anteil an dem insgesamt umzusetzenden Einsatz ausmachen, erfolgt typischerweise eine Spaltung dieser Kohlenwasserstoffe gemeinsam mit dem frisch zugeführten bzw. rückgeführten Ethan. Mit anderen Worten wird auf eine Bereitstellung separater Einheiten zum Dampfspalten verzichtet.

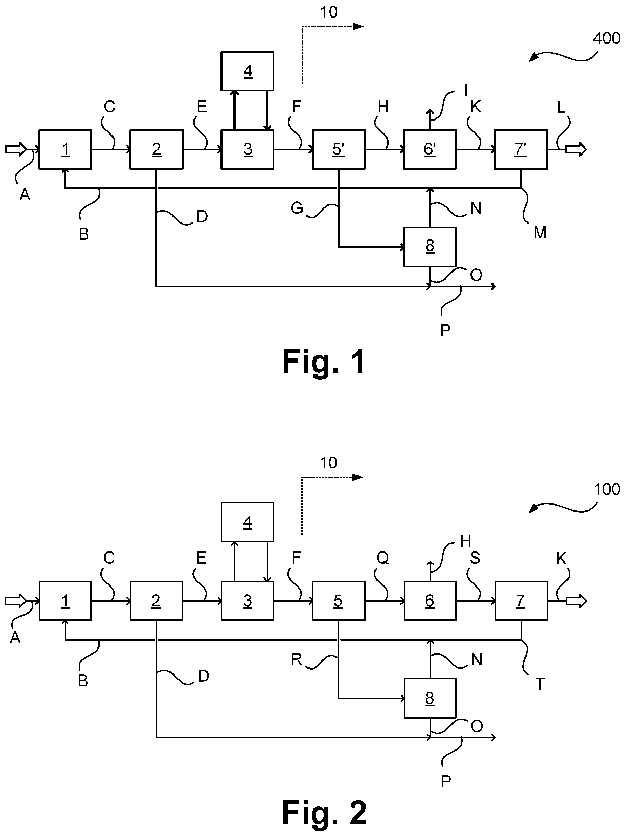

In Figur 1 ist ein entsprechendes Verfahren in Form eines schematischen Ablaufplans dargestellt und wird unten unter Bezugnahme auf Figur 1 im Detail erläutert. Wie dort angegeben, wird jedoch in diesem Verfahren eine Trennung vorgenommen, die aufwendiger ist, als sie für die erläuterten Zwecke erforderlich wäre.

Die vorliegende Erfindung stellt sich vor diesem Hintergrund die Aufgabe, ein entsprechendes Verfahren zu verbessern und trenntechnisch einfacher auszugestalten und hierdurch den Investitions- und/oder Betriebskosten zu reduzieren.

### Offenbarung der Erfindung

Vor diesen Hintergrund schlägt die vorliegende Erfindung ein Verfahren zur Gewinnung von Ethylen gemäß Anspruch 1 vor. Bevorzugte Ausgestaltungen sind Gegenstand der abhängigen Ansprüche sowie der nachfolgenden Beschreibung.

Vor der Erläuterung der Vorteile der vorliegenden Erfindung werden nachfolgend einige bei der Beschreibung der Erfindung verwendete Begriffe näher definiert. Komponentengemische können im hier verwendeten Sprachgebrauch reich oder arm an einer oder mehreren Komponenten sein, wobei der Begriff "reich" für einen Gehalt von wenigstens 75%, 80%, 90%, 95% oder 99% und der Begriff "arm" für einen Gehalt von höchstens 25%, 20%, 1 0%, 5% oder 1 % auf molarer, Gewichts- oder Volumenbasis stehen kann. Komponentengemische können im hier verwendeten Sprachgebrauch ferner angereichert oder abgereichert an einer oder mehreren Komponenten sein, wobei sich diese Begriffe auf einen entsprechenden Gehalt in einem anderen Komponentengemisch beziehen, unter Verwendung dessen das betrachtete Komponentengemisch gebildet wurde. Das betrachtete Komponentengemisch ist "angereichert", wenn es zumindest den 1,5-fachen, 2-fachen, 5-fachen, 10-fachen, 100-fachen oder 1.000-fachen Gehalt der bezeichneten Komponente(n) aufweist, und "abgereichert", wenn es höchstens den 0,75-fachen, 0,5-fachen, 0,1-fachen, 0,01-fachen oder 0,001-fachen Gehalt der bezeichneten Komponente(n) aufweist. Ein "überwiegend" eine oder mehrere Komponenten enthaltendes Komponentengemisch ist insbesondere reich an dieser bzw. diesen Komponenten im soeben erläuterten Sinn.

Ist hier davon die Rede, dass ein Komponentengemisch unter Verwendung eines anderen Komponentengemischs "gebildet" wird, sei darunter verstanden, dass das betrachtete Komponentengemisch zumindest einige der in dem anderen Komponentengemisch enthaltene oder diesen gebildete Komponenten aufweist. Ein Bilden eines Komponentengemischs aus einem anderen kann beispielsweise ein Abzweigen eines Teils des Komponentengemischs, ein Zuspeisen einer oder mehrerer weiterer Komponenten oder Komponentengemische, ein chemisches oder physikalisches Umsetzen zumindest einiger Komponenten, sowie ein Erwärmen, Abkühlen, Verdampfen, Kondensieren usw. umfassen. Ein "Bilden" eines Komponentengemischs aus einem anderen Komponentengemisch kann aber auch lediglich die Bereitstellung des anderen Komponentengemischs in geeigneter Form, beispielsweise in einem Behälter oder einer Leitung, umfassen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen. Jedoch bewegen sich derartige Drücke und Temperaturen typischerweise in bestimmten Bereichen, die beispielsweise ± 1 %, 5%, 1 0%, 20% oder 25% um einen Mittelwert liegen. Entsprechende Druckniveaus und Temperaturniveaus können in disjunkten Bereichen liegen oder in Bereichen, die einander überlappen. Dasselbe Druckniveau kann beispielsweise auch noch vorliegen, wenn es zu unvermeidlichen Druckverlusten kommt. Entsprechendes gilt für Temperaturniveaus. Bei den hier in bar angegebenen Druckniveaus handelt es sich um Absolutdrücke.

### Vorteile der Erfindung

Die vorliegende Erfindung schlägt insgesamt ein Verfahren zur Gewinnung von Ethylen vor, bei dem ein erstes Einsatzgas (Frischeinsatz) und ein zweites Einsatzgas (rückgeführtes Einsatzgas) einem Reaktor zugeführt und in diesem unter Erhalt eines Produktgemischs (Spaltgas) durch Dampfspalten bearbeitet werden. Das erste Einsatzgas kann insbesondere aus sogenanntem Rohethan mit fachüblichen Spezifikationen bestehen und insbesondere von der Anlagengrenze zugeführt werden. Das erste Einsatzgas weist mehr als 90, Gewichtsprozent, insbesondere mehr als 95 Gewichtsprozent, gesättigte Kohlenwasserstoffe sowie mehr als 80 Gewichtsprozent, insbesondere mehr als 85, 90 oder 95 Gewichtsprozent, Ethan auf. Ein Gehalt an Propan in dem ersten Einsatzgas liegt insbesondere bei bis zu 15 Gewichtsprozent, beispielsweise bei bis zu 10 oder bis zu 5 Gewichtsprozent.

Das erste Einsatzgas ist insbesondere arm an oder (im Wesentlichen) frei von schwereren Kohlenwasserstoffen, deren Gehalt insbesondere bei maximal 5 Gewichtsprozent oder 1 Gewichtsprozent liegen kann. Unter "schwereren Kohlenwasserstoffen" werden dabei hier insbesondere Kohlenwasserstoffe mit vier und mehr Kohlenstoffatomen verstanden.

Das erste Einsatzgas kann Methan in einem Gehalt von bis zu 5 Gewichtsprozent enthalten. Es ist insbesondere frei von oder zumindest arm an von Kohlendioxid und anderen Spurenkomponenten. Sämtliche ein "Einsatzgas" betreffende Erläuterungen beziehen sich auf den Frischeinsatz direkt am Reaktor, d.h. ein entsprechendes Einsatzgas kann ggf. bereits vorbehandelt, z.B. an CO2 abgereichert, sein.

Das Dampfspalten im Rahmen der vorliegenden Erfindung wird insbesondere zur Vermeidung der übermäßigen Bildung von Nebenprodukten mit einer mittleren oder geringen Ethankonversion von beispielsweise 65% oder weniger, 60% oder weniger, 55% oder weniger, 50% oder weniger oder 45% oder weniger und 10% oder mehr, 20% oder mehr oder 30% oder mehr durchgeführt. Daher verbleibt im Produktgemisch vergleichsweise viel Ethan. Weitere Parametereinstellungen beim Dampfspalten nimmt der Fachmann je nach Bedarf vor. Insbesondere werden entsprechende Einstellungen wie beispielsweise Dampfverdünnung und Reaktordruck derart gewählt, dass vergleichsweise viel Ethylen aus dem umgesetzten Ethan entsteht und vergleichsweise wenige Kohlenwasserstoffe mit drei oder mehr Kohlenstoffatomen entstehen.

Ist hier jeweils von "einem" Reaktor die Rede, versteht sich, dass anstelle lediglich eines Reaktors auch mehrere Reaktoren im seriellen oder parallelen Betrieb eingesetzt werden können, denen dann ein oder mehrere entsprechende Einsatzgase zugeführt werden können. Mehrere dieser Reaktoren ("Spaltöfen") können gleich oder unterschiedlich betrieben werden.

Im Rahmen der vorliegenden Erfindung wird das Produktgemisch, das dem Reaktor entnommen wird, oder ein Teil hiervon, unter Erhalt eines Folgegemischs, das Wasserstoff, Methan, Ethan, Ethylen sowie Kohlenwasserstoffe mit drei, vier und zumindest fünf Kohlenstoffatomen enthält und insbesondere (im Wesentlichen) aus diesen Komponenten besteht, einer Aufbereitung unterworfen. Die Aufbereitung kann dabei grundsätzlich in bekannter Weise erfolgen (siehe oben). Insbesondere kann eine derartige Aufbereitung auch eine Hydrierung von Acetylen sowie eine teilweise Hydrierung von insbesondere einfach und mehrfach ungesättigten Kohlenwasserstoffen mit drei Kohlenstoffatomen umfassen, um eine entsprechende Hydrierung bei der Rückführung von Komponenten zu vermeiden. Ein entsprechend gebildetes Folgegemisch oder ein Teil hiervon wird im Rahmen der vorliegenden Erfindung sodann einer Trennung unterworfen.

Die vorliegende Erfindung zeichnet sich dadurch aus, dass die Trennung einen Ethylenabtrennschritt umfasst, dem zumindest das Ethan, das Ethylen und die Kohlenwasserstoffe mit drei Kohlenstoffatomen aus dem Folgegemisch oder jeweils ein Teil hiervon in einem gemeinsamen Trenneinsatz ungetrennt voneinander zugeführt werden. Im Gegensatz zu bekannten Ethylenabtrennschritten, die im Stand der Technik typischerweise im Wesentlichen in Form einer Trennung von Ethan und Ethylen voneinander bestehen (in einem sogenannten C2-Splitter), wird im Rahmen der vorliegenden Erfindung in einem entsprechenden Trennschritt also Ethylen von einer verbleibenden Fraktion getrennt, die jedoch nicht nur Ethan, sondern auch wesentliche Teile der schwereren Kohlenwasserstoffe aus dem Produktgemisch enthält. Da im Rahmen der vorliegenden Erfindung aufgrund der Zusammensetzung des ersten Einsatzgases insgesamt vergleichsweise geringe Mengen an Kohlenwasserstoffen mit drei Kohlenstoffatomen gebildet werden und damit im Produktgemisch vorliegen, umfasst die verbleibende Fraktion insbesondere noch mehr als 50 Gewichtsprozent, insbesondere mehr als 60 Gewichtsprozent, mehr als 70 Gewichtsprozent, mehr als 80 Gewichtsprozent oder mehr als 85 Gewichtsprozent, und insbesondere bis zu 95 Gewichtsprozent oder bis zu 90 Gewichtsprozent Ethan und ansonsten schwerere Kohlenwasserstoffe, zumindest solche mit drei Kohlenstoffatomen. Die schwereren Kohlenwasserstoffe werden stromauf des Ethylenabtrennschritts jedoch nicht, wie in herkömmlichen Verfahren, abgetrennt. Dies gilt zumindest für die Kohlenwasserstoffe mit drei Kohlenstoffatomen, wie im Folgenden angegeben.

Je nach Ausgestaltung des erfindungsgemäßen Verfahrens kann dabei der Ethylenabtrennschritt auch mit Kohlenwasserstoffen mit vier und ggf. fünf Kohlenstoffatomen gespeist werden, wie jeweils nachfolgend noch im Detail erläutert. Das hier abgetrennte Ethylen bzw. eine leichte Fraktion aus dem Ethylenabtrennschritt kann als Ethylenprodukt aus dem Verfahren ausgeführt werden. Ein wesentlicher Aspekt der vorliegenden Erfindung besteht dabei in der ungetrennten Einspeisung der genannten Komponenten in den Ethylenabtrennschritt. Dies erleichtert die stromaufwärtige Trennung und trägt zu einer Verringerung des Trennaufwands bei.

Ist hier davon die Rede, dass zumindest das Ethan, das Ethylen und die Kohlenwasserstoffe mit drei Kohlenstoffatomen aus dem Folgegemisch oder jeweils ein Teil hiervon "ungetrennt" dem Ethylenabtrennschritt zugeführt werden, sei darunter verstanden, dass diese Komponenten zumindest zum Teil in einem durchgehenden Stoffstrom (von dem aber ein Teil oder bestimmte Komponenten, auch teilweise die die Kohlenwasserstoffe mit drei Kohlenstoffatomen, abgetrennt werden können) von dem Reaktor bis zum Ethylenabtrennschritt durchgeführt werden. Der Trenneinsatz für den Ethylenabtrennschritt kann je nach der spezifischen Ausgestaltung des Verfahrens insbesondere auch noch Kohlenwasserstoffe mit vier Kohlenstoffatomen oder Kohlenwasserstoffe mit vier und zumindest fünf Kohlenstoffatomen umfassen, die damit ebenfalls ungetrennt dem Ethylenabtrennschritt zugeführt werden.

In dem Ethylenabtrennschritt werden die erwähnte leichte Fraktion, die mehr als 95 Molprozent, insbesondere mehr als 99 Molprozent, Ethylen enthält und insbesondere (im Wesentlichen) aus Ethylen bestehen kann, und eine schwere Fraktion, die zumindest einen Teil des Ethans aus dem Trenneinsatz (S, V, X) und mindestens 15 Gewichtsprozent der Kohlenwasserstoffe mit drei und vier Kohlenstoffatomen (ggf. auch schwerere Kohlenwasserstoffe), gebildet. Die schwere Fraktion kann insbesondere mehr als 20 Gewichtsprozent, mehr als 30 Gewichtsprozent, mehr als 40 Gewichtsprozent, mehr als 50 Gewichtsprozent, mehr als 60 Gewichtsprozent, mehr als 70 Gewichtsprozent, mehr als 80 Gewichtsprozent oder mehr als 90 Gewichtsprozent der Kohlenwasserstoffe mit drei und vier Kohlenstoffatomen (ggf. auch schwerere Kohlenwasserstoffe) aufweisen, wobei die zuletzt genannten Angaben jeweils auch Obergrenzen entsprechender Wertebereiche angeben können. Die Werte ergeben sich durch die vergleichsweise starke Verdünnung dieser Komponenten mit Ethan. Die schwere Fraktion kann (im Wesentlichen) frei von Ethylen sein. Die schwere Fraktion aus dem Ethylenabtrennschritt oder ein Teil hiervon wird (direkt oder unter Abtrennung ggf. enthaltener schwererer Komponenten) zur Bildung des zweiten Einsatzgases verwendet.

Zur Verringerung des Trennaufwands kann im Rahmen der vorliegenden Erfindung insbesondere eine "weiche" bzw. "unscharfe" Deethanisierung erfolgen, in der jeweils nicht, wie aus dem Stand der Technik bekannt, eine Fraktion gebildet wird, die Ethan und Ethylen enthält, aber (im Wesentlichen) frei von anderen, insbesondere von schwereren, Komponenten ist. Diese wird mit einer Demethanisiserung kombiniert. Es kann jedoch auch nur eine Demethanisierung erfolgen.

So kann im Rahmen der vorliegenden Erfindung der Trenneinsatz, der dem Ethylenabtrennschritt zugeführt wird, unter Verwendung eines ersten Vortrennschritts und eines zweiten Vortrennschritts gebildet werden, wobei dem ersten Vortrennschritt das Folgegemisch oder dessen der Trennung unterworfener Teil in unveränderter Zusammensetzung zugeführt wird, wobei in dem ersten Vortrennschritt eine leichte Fraktion und eine schwere Fraktion gebildet werden, wobei die leichte Fraktion aus dem ersten Vortrennschritt oder ein Teil hiervon dem zweiten Vortrennschritt zugeführt wird, wobei in dem zweiten Vortrennschritt eine leichte Fraktion und eine schwere Fraktion gebildet werden, und wobei die schwere Fraktion aus dem zweiten Vortrennschritt oder ein Teil hiervon als der Trenneinsatz oder ein Teil des Trenneinsatzes verwendet wird, der dem Ethylentrennabschritt zugeführt wird. Der erste Vortrennschritt entspricht dabei der bereits erwähnten unscharfen Deethanisierung, der zweite Vortrennschritt einer (insoweit dem Üblichen entsprechenden) Demethanisierung.

Die Trenngrenzen in der erfindungsgemäß eingesetzten unscharfen Deethanisierung können dabei unterschiedlich gesetzt werden. So kann in einer ersten Alternative die leichte Fraktion aus dem ersten Vortrennschritt insgesamt weniger als 1 Molprozent an Kohlenwasserstoffen mit vier und zumindest fünf Kohlenstoffatomen und im Rest Methan, Ethan, Ethylen und Kohlenwasserstoffe mit drei Kohlenstoffatomen enthalten. Die schwere Fraktion aus dem ersten Vortrennschritt kann in diesem Fall insgesamt weniger als 1 Molprozent Wasserstoff, Methan und Ethylen und im Rest Ethan, Kohlenwasserstoffe mit drei, vier und zumindest fünf Kohlenstoffatomen enthalten. Die Aufgabe dieses Trennschritts in der ersten Alternative ist insbesondere die Trennung von Ethylen und Kohlenwasserstoffen mit vier und zumindest fünf Kohlenstoffatomen, wohingegen die Kohlenwasserstoffe mit drei Kohlenstoffatomen in beiden Fraktionen enthalten sind. In dieser ersten Alternative enthält ferner die leichte Fraktion aus dem zweiten Vortrennschritt, also aus der Demethanisierung, insgesamt mehr als 99 Molprozent Methan und Wasserstoff, und die schwere Fraktion aus dem zweiten Vortrennschritt enthält insgesamt weniger als 1 Molprozent Methan und Wasserstoff und im Rest Ethan, Ethylen und Kohlenwasserstoffe mit drei Kohlenstoffatomen.

In einer zweiten Alternative kann die Trenngrenze in der unscharfen Deethanisierung derart gesetzt werden, dass die leichte Fraktion aus dem ersten Vortrennschritt insgesamt weniger als 1 Molprozent an Kohlenwasserstoffen mit zumindest fünf Kohlenstoffatomen und im Rest Methan, Ethan, Ethylen und Kohlenwasserstoffe mit drei und vier Kohlenstoffatomen, und die schwere Fraktion aus dem ersten Vortrennschritt insgesamt weniger als 1 Molprozent an Wasserstoff, Methan und Ethylen und im Rest Ethan sowie Kohlenwasserstoffe mit drei, vier und zumindest fünf Kohlenstoffatomen enthält. Die Aufgabe dieses Trennschritts in der zweiten Alternative ist insbesondere die Trennung von Ethylen und Kohlenwasserstoffen mit zumindest fünf Kohlenstoffatomen. In dieser zweiten Alternative enthält die leichte Fraktion aus dem zweiten Vortrennschritt, also der Demethanisierung, grundsätzlich wie oben insgesamt mehr als 99 Molprozent Methan und Wasserstoff. Die schwere Fraktion aus dem zweiten Vortrennschritt enthält auch hier insgesamt weniger als 1 Molprozent Methan und Wasserstoff, jedoch im Rest Ethan, Ethylen und Kohlenwasserstoffe mit drei und vier Kohlenstoffatomen.

In beiden der zuvor erläuterten Alternativen kann die schwere Fraktion aus dem ersten Vortrennschritt oder ein Teil hiervon jeweils einem weiteren Trennschritt unterworfen werden, in dem wiederum eine leichte Fraktion und eine schwere Fraktion, gebildet werden. Die leichte Fraktion enthält hierbei entweder weniger als 1 Molprozent an Kohlenwasserstoffen mit zumindest sechs Kohlenstoffatomen und im Rest Kohlenwasserstoffe mit drei, vier und fünf Kohlenstoffatomen oder weniger als 1 Molprozent an Kohlenwasserstoffen mit zumindest fünf Kohlenstoffatomen und im Rest Kohlenwasserstoffe mit drei und vier Kohlenstoffatomen. Die schwere Fraktion kann in diesem Fall entweder, gemäß der soeben angegebenen ersten Alternative für die Zusammensetzung der leichten Fraktion, überwiegend oder ausschließlich Kohlenwasserstoffe mit zumindest sechs Kohlenstoffatomen oder, gemäß der soeben angegebenen zweiten Alternative für die Zusammensetzung der leichten Fraktion, überwiegend oder ausschließlich Kohlenwasserstoffe mit zumindest fünf Kohlenstoffatomen enthalten.

Aufgabe des zuletzt erläuterten Trennschritts ist die Erzeugung eines geeigneten Recycles. Hierbei sollten zumindest Kohlenwasserstoffe mit sechs Kohlenstoffatomen entfernt werden, ggf. auch Kohlenwasserstoffe mit fünf Kohlenstoffatomen. Es handelt sich bei diesem weiteren Trennschritt also in letzterem Fall um einen typischen Debutanisierungsschritt, wie er grundsätzlich aus dem Stand der Technik bekannt ist.

Gemäß einer dritten Alternative kann der Trenneinsatz, der dem Ethylenabtrennschritt zugeführt wird, jedoch auch unter Verwendung nur eines einzigen Vortrennschritts gebildet werden, dem das Folgegemisch unveränderter Zusammensetzung zugeführt wird. Hierbei handelt es sich ebenfalls um eine Demethanisierung. In diesem einzigen Vortrennschritt wird also eine leichte Fraktion, die insgesamt mehr als 99 Molprozent Methan und Wasserstoff enthält, gebildet. Aufgrund der zusätzlich zugeführten Kohlenwasserstoffe enthält eine schwere Fraktion, die in dem einzigen Vortrennschritt gebildet wird, jedoch insgesamt weniger als 1 Molprozent Methan und Wasserstoff und im Rest Ethan, Ethylen und Kohlenwasserstoffe mit drei, vier und zumindest fünf Kohlenstoffatomen. Diese schwere Fraktion aus dem einzigen Vortrennschritt oder ein Teil hiervon wird als der Trenneinsatz oder als ein Teil des Trenneinsatzes verwendet, der dem Ethylenabtrennschritt zugeführt wird.

Wird anstelle eines ersten und zweiten Vortrennschritts, wie in der soeben erläuterten dritten Alternative, ein einziger Vortrennschritt eingesetzt, wird vorteilhafterweise zumindest ein Teil der schweren Fraktion, der nach dem Ethylenabtrennschritt verbleibt, einem weiteren Trennschritt zugeführt, in dem eine leichte Fraktion und eine schwere Fraktion gebildet werden. Die leichte Fraktion enthält hierbei entweder weniger als 1 Molprozent an Kohlenwasserstoffen mit zumindest sechs Kohlenstoffatomen und im Rest Kohlenwasserstoffe mit drei, vier und fünf Kohlenstoffatomen oder weniger als 1 Molprozent an Kohlenwasserstoffen mit zumindest fünf Kohlenstoffatomen und im Rest Kohlenwasserstoffe mit drei und vier Kohlenstoffatomen. Die schwere Fraktion kann in diesem Fall entweder, gemäß der soeben angegebenen ersten Alternative für die Zusammensetzung der leichten Fraktion, überwiegend oder ausschließlich Kohlenwasserstoffe mit zumindest sechs Kohlenstoffatomen oder, gemäß der soeben angegebenen zweiten Alternative für die Zusammensetzung der leichten Fraktion, überwiegend oder ausschließlich Kohlenwasserstoffe mit zumindest fünf Kohlenstoffatomen enthalten.

Auch hier besteht die Aufgabe des zuletzt erläuterten Trennschritts in der Erzeugung eines geeigneten Recycles, bei der zumindest Kohlenwasserstoffe mit sechs Kohlenstoffatomen, ggf. auch Kohlenwasserstoffe mit fünf Kohlenstoffatomen, entfernt werden. Es handelt sich bei diesem weiteren Trennschritt in letzterem Fall also wieder um einen typischen Debutanisierungsschritt, wie er grundsätzlich aus dem Stand der Technik bekannt ist.

Es versteht sich, dass in jedem der Fälle, in denen wie zuvor und nachfolgend erläutert Kohlenwasserstoffe mit zumindest sechs Kohlenstoffatomen in eine Fraktion überführt werden, diese Kohlenwasserstoffe in dem Produktgemisch enthalten sind.

In sämtlichen Fällen, in denen ein zuvor als "weiterer Trennschritt" bezeichneter Trennschritt eingesetzt wird, wird dessen leichte Fraktion oder ein Teil hiervon zur Bildung des zweiten Einsatzgases verwendet, da diese arm an oder frei von Kohlenwasserstoffen mit sechs und ggf. mehr Kohlenstoffatomen und ggf. auch an fünf Kohlenstoffatomen ist und Kohlenwasserstoffe enthält, die sich zur Rückführung in der Dampfspaltung eignen. Dieses ist insbesondere dann der Fall, wenn die mehrfach erläuterte Aufbereitung des Spaltgases eine Hydrierung umfasst. Die leichte Fraktion aus dem weiteren Trennschritt kann dabei in der ersten und zweiten Alternative mit der schweren Fraktion aus dem Ethylenabtrennschritt kombiniert werden.

Die schwere Fraktion aus dem weiteren Trennschritt wird hingegen typischerweise zusammen mit einer Fraktion aus der Aufbereitung, die Kohlenwasserstoffe mit zumindest fünf oder zumindest sechs Kohlenstoffatomen enthält, aus dem Verfahren ausgeführt. Es handelt sich hierbei um sogenanntes Pyrolysebenzin, das nach Aufbereitung in an sich bekannter Weise beispielsweise als Kraftstoff oder zur Gewinnung von Aromaten verwendet werden kann.

Die zuvor erläuterten drei Alternativen der vorliegenden Erfindung (mit erstem und zweitem Vortrennschritt mit unterschiedlichen Trenngrenzen im ersten Vortrennschritt und die Alternative ohne ersten Vortrennschritt) weisen jeweils unterschiedliche Vorteile hinsichtlich der Erstellungs- und Betriebskosten auf, welche nachfolgend zusammengefasst sind. Der Fachmann wählt daher die zuvor erläuterten Alternativen je nach den gestellten Anforderungen aus.

Im Rahmen der ersten Alternative kann eine Verringerung der erforderlichen Wellenleistung um 3,6 MW, in der zweiten Alternative um 6,3 MW und in der dritten Alternative um 6,9 MW (hier und nachfolgend bezieht sich die "Verringerung" auf ein nicht erfindungsgemäßes Verfahren, wie es in Figur 1 veranschaulicht ist, erzielt werden. Dies ist auf die unschärfere bzw. wegfallende Trennung in dem Deethanizer zurückzuführen. Es ergibt sich ferner jeweils eine Reduzierung in der Menge an erforderlichem Niederdruckdampf (ausgedrückt in der zu dessen Bereitstellung erforderlichen Energiemenge) um 10,8 MW (erste Alternative), 13 MW (zweite Alternative) bzw. 16 MW (dritte Alternative 3). Dies ist auf die verringerte bzw. wegfallende Heizleistung in der Deethanisierung zurückzuführen.

In der zweiten Alternative kann die Deethanisierung auf deutlich geringerem Druck als in der ersten Alternative und in der nicht erfindungsgemäßen Ausführungsform erfolgen, was neben der geringeren Verdichterleistung auch insbesondere Materialeinsparungen zur Folge hat. Entsprechendes gilt für die Bodenzahl in der Deethanisierung, die in der zweiten Alternative ebenfalls verringert werden kann. In der dritten Alternative ist es ggf. vorteilhaft, die Debutanisierung mit einem zusätzlichen Niederdruckabsorber auszustatten.

In der dritten Alternative ist insbesondere aufgrund der wegfallenden Deethanisierung eine Verringerung der Rippenplattenwärmetauscherfläche um ca. 600 kW/K möglich. Eine Verringerung der Block-in-Shell-Wärmetauscherfläche beträgt in der ersten Alternative ca. 750 kW/K, in der zweiten Alternative ca. 1 450 kW/K und in der dritten Alternative ca. 1 800 kW/K. Die Rohrbündelwärmetauscherfläche kann in der ersten Alternative um ca. 2 600 kW/K, in der zweiten Alternative um ca. 4 300 kW/K und in der dritten Alternative um ca. 5 700 kW/K reduziert werden.

Die Erfindung wird nachfolgend unter Bezugnahme auf die beigefügten Zeichnungen näher erläutert, welche Ausführungsformen der vorliegenden Erfindung im Vergleich mit einer nicht erfindungsgemäßen Ausführungsform veranschaulichen.

### Kurze Beschreibung der Zeichnungen

Figur 1 veranschaulicht ein Verfahren gemäß einer nicht erfindungsgemäßen Ausführungsform.
Figur 2 veranschaulicht ein Verfahren gemäß einer Ausführungsform der Erfindung.
Figur 3 veranschaulicht ein Verfahren gemäß einer Ausführungsform der Erfindung.
Figur 4 veranschaulicht ein Verfahren gemäß einer Ausführungsform der Erfindung.

### Ausführliche Beschreibung der Zeichnungen

In den nachfolgenden Figuren sind einander baulich oder funktionell entsprechende Elemente jeweils mit identischen Bezugszeichen angegeben. Entsprechendes gilt für die mit Großbuchstaben bezeichneten Stoffströme. Es versteht sich jeweils, dass entsprechende Komponenten andere bauliche Ausgestaltungen erfahren bzw. entsprechende Stoffströme teilweise anders zusammengesetzt sein können, ohne hierbei jeweils abweichend bezeichnet zu werden.

In Figur 1 ist ein Verfahren zur Gewinnung von Ethylen gemäß einer nicht erfindungsgemäßen Ausführungsform in Form eines schematischen Verfahrensdiagramms veranschaulicht und insgesamt mit 400 bezeichnet.

In dem Verfahren 400 wird einem Reaktor 1 von der Anlagengrenze (wie durch ein Pfeilsymbol veranschaulicht) ein Einsatzgas A zugeführt. Dieses Einsatzgas A wurde zuvor und wird im Folgenden jeweils als "erstes" Einsatzgas bezeichnet. Es handelt sich dabei beispielsweise im Wesentlichen um reines Ethylen. Zu möglichen Ethylengehalten eines entsprechenden ersten Einsatzgases sei auf die obigen Erläuterungen verwiesen. Ferner wird dem Reaktor ein weiteres Einsatzgas, zuvor und nachfolgend als "zweites" Einsatzgas bezeichnet, zugeführt. Bei diesem zweiten Einsatzgas handelt es sich um ein aus dem Verfahren 400 zurückgeführtes Gasgemisch, das insbesondere Ethan und Kohlenwasserstoffe mit drei und vier Kohlenstoffatomen enthält. Zur Bildung des zweiten Einsatzgases wird auf die nachfolgenden Erläuterungen verwiesen.

Das erste Einsatzgas A und das zweite Einsatzgas B werden dem Reaktor 1 zugeführt und dort mit Dampf (nicht veranschaulicht) durch Dampfspalten bearbeitet. Auf diese Weise wird ein Produktgemisch C erhalten, das fachüblich auch als Spaltgas bezeichnet wird. Das Produktgemisch C enthält neben Produkten und Nebenprodukten des Dampfspaltens insbesondere auch nicht umgesetzte Edukte, im vorliegenden Fall insbesondere Ethan, sowie Wasser aus dem zugegebenen Wasserdampf. Das Produktgemisch C wird zur Entfernung entsprechender unerwünschter Komponenten daher einer Aufbereitung mit den Verfahrensschritten 2 bis 4 und einer insgesamt mit 10 zusammengefassten Trennung zugeführt.

Die Aufbereitung umfasst dabei zunächst einen Wasserquench zur Kühlung und Prozessdampfkondensation sowie eine Verdichtung in einem typischerweise mehrstufigen Verdichter in einem Verfahrensschritt 2. Hierbei können sich bereits Kohlenwasserstoffe mit fünf und mehr Kohlenstoffatomen aus dem Produktgemisch C, also Komponenten des sogenannten Pyrolysebenzins, abscheiden. Entsprechende Komponenten können in Form eines Stoffstroms D aus dem Verfahrensschritt 2 abgezogen werden. Auch eine Sauergasentfernung kann erfolgen. Ein verdichtetes und entsprechend von zumindest einem Teil der Kohlenwasserstoffe mit fünf Kohlenstoffatomen befreites Gasgemisch, das nun mit E bezeichnet ist, kann nun einer Vorkühlung und Trocknung 3 zugeführt werden. Im Zuge dieser Vorkühlung und Trocknung kann auch eine Hydrierung 4, die sogenannte Rohgashydrierung, durchgeführt werden, bei dem insbesondere Acetylen zu Ethylen sowie einfach und mehrfach ungesättigte Kohlenwasserstoffe mit drei und mehr Kohlenstoffatomen und schwerere Komponenten teilweise zu weniger ungesättigten Komponenten umgesetzt werden. Eine Hydrierung kann auch in der nachfolgend beschriebenen Trennung 10 erfolgen, was jedoch den Nachteil hätte, dass in den meisten Ausgestaltungen Acetylen und zum Reaktor zurückzuführende, zunächst noch ungesättigte Kohlenwasserstoffe mit drei und vier Kohlenstoffatomen gesondert hydriert werden müssen (nicht weiter beschrieben). Ein in den Verfahrensschritten 2 bis 4 bearbeitetes Gasgemisch F, zuvor und nachfolgend als "Folgegemisch" bezeichnet, wird der Trennung 10 zugeführt.

Die Trennung 10 umfasst im dargestellten Beispiel zunächst einen Dethanisierungsschritt 5', zuvor und nachfolgend auch als "erster Vortrennschritt" bezeichnet, in dem eine leichte Fraktion H, die im Wesentlichen Ethan, Ethylen und leichtersiedende Verbindungen enthält, und eine schwere Fraktion G, die im Wesentlichen Kohlenwasserstoffe mit drei und mehr Kohlenstoffatomen enthält, gebildet werden. Erstere wird einer Demethanisierung 6, zuvor und nachfolgend auch als "zweiter Vortrennschritt" bezeichnet, zugeführt, letztere einer Debutanisierung 8, zuvor und nachfolgend auch als "weiterer Trennschritt" bezeichnet.

In dem zweiten Vortrennschritt 6 wird die leichte Fraktion H aus dem ersten Vortrennschritt 5', die, wie erwähnt, im Wesentlichen Ethan, Ethylen und leichtersiedende Verbindungen enthält, von den leichtersiedenden Verbindungen befreit. Auf diese Weise wird eine leichte Fraktion I gebildet, die im Wesentlichen Methan und Wasserstoff enthält, und die aus dem Verfahren 100 abgezogen und/oder beispielsweise zur Befeuerung des Reaktors 1 verwendet werden kann. Auch eine Rückgewinnung von Wasserstoff aus einem entsprechenden Gasgemisch ist möglich. Hierbei sei darauf hingewiesen, dass in weiteren Ausgestaltungen der Vortrennschritt 5' (bzw. Vortrennschritt 5 und 5" in den erfindungsgemäßen Folgebeispielen) und Vortrennschritt 6 in ihrer Reihenfolge vertauscht werden können. Eine schwere Fraktion K aus dem zweiten Vortrennschritt 6, ein von Methan und Wasserstoff befreites Gasgemisch, das in dem Verfahren 400 noch im Wesentlichen Ethan und Ethylen enthält, wird nachfolgend einem Ethylenabtrennnschritt 7', der hier einer klassischen C2-Trennung entspricht, zugeführt, wo eine im Wesentlichen Ethylen enthaltende leichte Fraktion L und eine im Wesentlichen Ethan enthaltende schwere Fraktion M gebildet werden. Erstere kann in Form eines Stoffstroms L an die mit dem Pfeilsymbol veranschaulichte Anlagengrenze abgegeben werden, letztere wird ein in Form eines Stoffstroms M zur Bildung des zweiten Einsatzgases B verwendet.

In dem weiteren Trennschritt 8 wird aus der schweren Fraktion G aus dem ersten Vortrennschritt eine leichte Fraktion N, die im Wesentlichen Kohlenwasserstoffe mit drei und vier (und ggf. fünf, siehe obige Erläuterungen) Kohlenstoffatomen enthält, und eine schwere Fraktion O, die im Wesentlichen Kohlenwasserstoffe mit fünf und ggf. mehr Kohlenstoffatomen enthält, gebildet. Erstere wird mit der schweren Fraktion M aus dem Ethylenabtrennschritt vereinigt und damit zur Bildung des zweiten Einsatzgases B verwendet. Die schwere Fraktion O aus dem weiteren Trennschritt 8 wird mit dem Stoffstrom D vereinigt und in Form eines Pyrolysebenzinstroms P ausgeführt.

Wie bereits zuvor erwähnt, wird in dem in Figur 1 veranschaulichten Verfahren 400 eine an sich überflüssige vollständige Trennung von Kohlenwasserstoffen mit drei und vier Kohlenstoffatomen von Ethan vorgenommen. Insbesondere wird dabei in der Deethanisierung 5 ein überflüssiger Trennaufwand betrieben. Die vorliegende Erfindung schlägt daher in unterschiedlichen Ausgestaltungen ein Verfahren vor, in denen ein entsprechender Trennaufwand reduziert ist.

In Figur 2 ist ein Verfahren gemäß einer Ausführungsform der vorliegenden Erfindung veranschaulicht und insgesamt mit 100 bezeichnet. Diese Ausführungsform entspricht der oben mehrfach erwähnten "ersten Alternative".

Das Verfahren 100 gemäß Figur 2 unterscheidet sich von dem in Figur 1 veranschaulichten nicht erfindungsgemäßen Verfahren 400 hinsichtlich der Verfahrensschritte 1 bis 4 nicht notwendigerweise. Bezüglich dieser Verfahrensschritte wird daher auf die obigen Erläuterungen verwiesen. In dem Verfahren 100 gemäß Figur 2 wird jedoch eine von dem Verfahren 400 gemäß Figur 1 abweichende Dethanisierung als erster Trennschritt vorgenommen, der daher hier abweichend mit 5 bezeichnet ist. Im Gegensatz zu einem im Wesentlichen Ethan, Ethylen und leichtere Komponenten enthaltenden Gasgemisch (vgl. leichte Fraktion H gemäß Figur 1) einerseits und einem im Wesentlichen Kohlenwasserstoffe mit drei und mehr Kohlenstoffatomen enthaltenden Gasgemisch andererseits (vgl. leichte Fraktion G gemäß Figur 1) werden hier eine leichte Fraktion Q, die neben Ethan, Ethylen und leichteren Komponenten auch einen Teil der Kohlenwasserstoffe mit drei Kohlenstoffatomen aus dem dem ersten Vortrennschritt 5 zugeführten Folgegemisch F enthält, und eine schwere Fraktion R, die nur einen Teil der Kohlenwasserstoffe mit drei Kohlenstoffatomen und ansonsten die schwereren Kohlenwasserstoffe aus dem Folgegemisch F sowie ggf. Ethan enthält, gebildet. Die leichte Fraktion Q ist dabei insbesondere im Wesentlichen frei von Kohlenwasserstoffen mit vier und mehr Kohlenstoffatomen. Umgekehrt ist die schwere Fraktion R abgezogene Fraktion arm an oder frei von Ethylen und leichter siedenden Komponenten.

Die leichte Fraktion Q wird auch hier dem zweiten Vortrennschritt 6 zugeführt, in dem eine im Wesentlichen Methan und Wasserstoff enthaltende leichte Fraktion H gebildet wird. Eine verbleibende schwere Fraktion S enthält jedoch im Gegensatz zu der schweren Fraktion K aus dem zweiten Vortrennschritt 6 des Verfahrens 400 gemäß Figur 1 neben Ethan und Ethylen auch Kohlenwasserstoffe mit drei Kohlenstoffatomen. Es wird einem Ethylenabtrennschritt 7 zugeführt. Im Gegensatz zu dem Ethylenabtrennschritt 7" des Verfahrens 400 gemäß Figur 1 wird hier zwar auch eine im Wesentlichen Ethylen enthaltende leichte Fraktion K gebildet, jedoch verbleibt als schwere Fraktion T ein Gasgemisch aus im Wesentlichen Ethan und Kohlenwasserstoffen mit drei Kohlenstoffatomen. Dieses kann insbesondere im Sumpf einer in dem Ethylenabtrennschritt 7 verwendeten Rektifikationskolonne anfallen. Die schwere Fraktion T aus dem Ethylenabtrennschritt 7 wird in der veranschaulichten Weise bei der Bildung des zweiten Einsatzgases B verwendet.

Der weitere Trennschritt 8 gemäß dem Verfahren 100 entspricht im Wesentlichen dem Trennschritt 8 gemäß dem Verfahren 400 gemäß Figur 1, wobei hier jedoch in die leichte Fraktion N des weiteren Trennschritts 8 nur jene Kohlenwasserstoffe mit drei Kohlenstoffatomen übergehen, die nicht bereits über die leichte Fraktion Q und aus dem ersten Vortrennschritt 5 und die schwere Fraktion S aus dem zweiten Vortrennschritt 6 in die schwere Fraktion T aus dem Ethylenabtrennschritt 7 übergegangen sind.

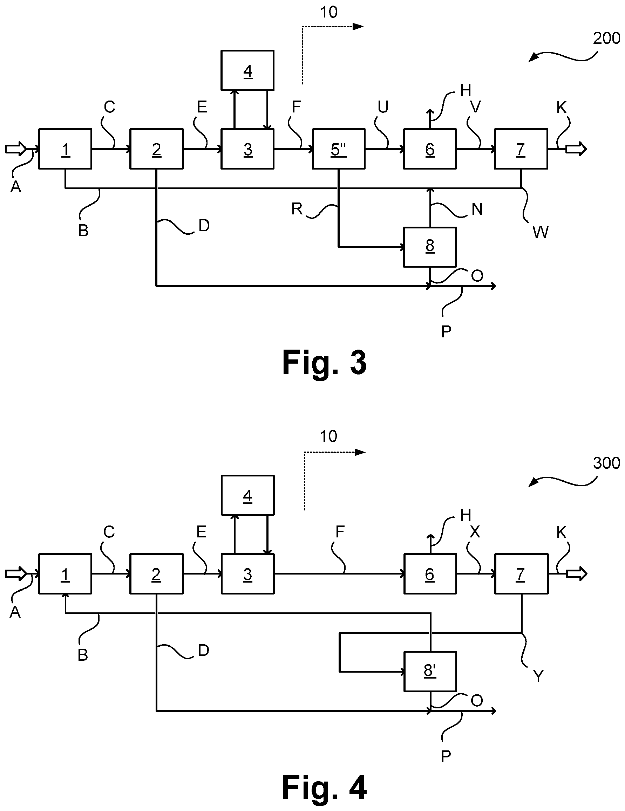

In Figur 3 ist ein Verfahren gemäß einer weiteren Ausführungsform der vorliegenden Erfindung veranschaulicht und insgesamt mit 200 bezeichnet. Das Verfahren 200 entspricht der zuvor mehrfach erwähnten "zweiten Alternative". Es unterscheidet sich von dem Verfahren 100 gemäß Figur 2 im Wesentlichen durch die abweichende Ausführung des ersten Vortrennschritts 5. Der erste Vortrennschritt 5 ist daher in dem Verfahren 200 gemäß Figur 3 mit 5" bezeichnet.

Im Gegensatz zu dem ersten Vortrennschritt 5 gemäß dem Verfahren 100 wird in dem ersten Vortrennschritt 5" gemäß dem Verfahren 200 eine leichte Fraktion U gebildet, die neben Ethan, Ethylen und den leichter siedenden Komponenten nicht nur einen Teil der Kohlenwasserstoffe mit drei Kohlenstoffatomen, sondern auch einen Teil der Kohlenwasserstoffe mit vier Kohlenstoffatomen aus dem Folgegemisch F enthält. Diese wird zum zweiten Vortrennschritt 6 geführt. Während eine in dem zweiten Vortrennschritt 6 gebildete leichte Fraktion H auch hier überwiegend oder ausschließlich Methan und Wasserstoff enthält, enthält die verbleibende schwere Fraktion V Ethan, Ethylen sowie Kohlenwasserstoffe mit drei und vier Kohlenstoffatomen. In dem Ethylenabtrennschritt, der hier wie zuvor mit 7 bezeichnet ist, werden eine im Wesentlichen Ethylen enthaltende leichte Fraktion K sowie eine im Wesentlichen Ethan, Kohlenwasserstoffe mit drei und Kohlenwasserstoffe mit vier Kohlenstoffatomen enthaltende schwere Fraktion W gebildet.

Eine in dem ersten Vortrennschritt 5" gebildete schwere Fraktion R enthält die über die Fraktionen U und V in die Fraktion W übergehenden Kohlenwasserstoffen mit drei und vier Kohlenstoffatomen nicht, so dass diese auch nicht in die leichte Fraktion N des weiteren Trennschritts 8 übergehen.

Figur 4 veranschaulicht eine weitere Ausführungsform der vorliegenden Erfindung, die insgesamt mit 300 bezeichnet ist. Das Verfahren 300 entspricht der zuvor mehrfach erwähnten "zweiten Alternative". Wie anhand des Verfahrens 300 in Figur 4 veranschaulicht, kann in einer erfindungsgemäßen Ausführungsform auch vollständig auf einen ersten Vortrennschritt 5', 5 oder 5" (siehe vorherige Figuren 1 bis 3) verzichtet werden. In diesem Fall wird der gesamte Stoffstrom F, also das erläuterte Folgegemisch, der Demethanisierung 6, also einem einzigen Vortrennschritt, zugeführt. Eine hier erhaltene schwere Fraktion X enthält damit neben Ethan und Ethylen auch Kohlenwasserstoffe mit drei, vier und zumindest fünf Kohlenstoffatomen. Diese Fraktion X wird dem Ethylenabtrennschritt 7 zugeführt, in dem auch hier die überwiegend oder ausschließlich Ethylen enthaltende leichte Fraktion K gebildet und abgezogen werden kann. Eine verbleibende schwere Fraktion Y kann einem weiteren Trennschritt zugeführt werden, der vergleichbar mit dem Trennschritt 8 gemäß den vorliegenden Figuren ausgestaltet sein kann und daher mit 8' bezeichnet ist. Die in diesem Trennschritt 8' gebildete leichte Fraktion enthält Ethan und Kohlenwasserstoffe mit drei und vier (und ggf. fünf) Kohlenstoffatomen. Sie wird daher direkt als das zweite Einsatzgas B verwendet. Die Zusammensetzung der schweren Fraktion O unterscheidet sich nicht von jener gemäß den zuvor erläuterten Ausgestaltungen der Erfindung.

## Patentansprüche

1. Verfahren (100, 200, 300) zur Gewinnung von Ethylen, bei dem ein erstes Einsatzgas (A) und ein zweites Einsatzgas (B) einem Reaktor (1) zugeführt und in diesem unter Erhalt eines Produktgemischs (C) durch Dampfspalten bearbeitet werden, wobei das erste Einsatzgas (A) mehr als 90 Gewichtsprozent gesättigte Kohlenwasserstoffe und mehr als 80 Gewichtsprozent Ethan auweist, und wobei das Produktgemisch (C) oder ein Teil hiervon unter Erhalt eines Folgegemischs (F), das Wasserstoff, Methan, Ethan, Ethylen sowie Kohlenwasserstoffe mit drei, vier und zumindest fünf Kohlenstoffatomen enthält, einer Aufbereitung (2, 3, 4) und das Folgegemisch (F) oder ein Teil hiervon einer Trennung (10) unterworfen wird, **dadurch gekennzeichnet, dass** die Trennung (10) einen Ethylenabtrennschritt (7) umfasst, dem zumindest das Ethan, das Ethylen und die Kohlenwasserstoffe mit drei Kohlenstoffatomen aus dem Folgegemisch (F) oder jeweils ein Teil hiervon in einem gemeinsamen Trenneinsatz (S, V, X) ungetrennt voneinander zugeführt werden, wobei in dem in dem Ethylenabtrennschritt (7) eine leichte Fraktion (K), die mehr als 95 Molprozent Ethylen enthält, und eine schwere Fraktion (T, W, Y), die zumindest einen Teil des Ethans aus dem Trenneinsatz (S, V, X) und mindestens 15 Gewichtsprozent der Kohlenwasserstoffe mit drei und vier Kohlenstoffatomen aus dem Trenneinsatz (S, V, X), gebildet werden, und wobei das schwere Trennprodukt (T, W, Y) aus dem Ethylenabtrennschritt (7) oder ein Teil hiervon als Teil oder zur Bildung des zweiten Einsatzgases (B) verwendet wird.

2. Verfahren (100, 200) nach Anspruch 1, bei dem der Trenneinsatz (S, V), der dem Ethylenabtrennschritt (7) zugeführt wird, unter Verwendung eines ersten Vortrennschritts (5, 5") und eines zweiten Vortrennschritts (6) gebildet wird, wobei dem ersten Vortrennschritt (5, 5") das Folgegemisch (F) oder dessen der Trennung (10) unterworfener Teil in unveränderter Zusammensetzung zugeführt wird, wobei in dem ersten Vortrennschritt (5, 5") eine leichte Fraktion (Q, U) und eine schwere Fraktion (R) gebildet werden, wobei die leichte Fraktion (Q, U) aus dem ersten Vortrennschritt (5, 5") oder ein Teil hiervon dem zweiten Vortrennschritt (6) zugeführt wird, wobei in dem zweiten Vortrennschritt (6) eine leichte Fraktion (H) und eine schwere Fraktion (S, V) gebildet werden, und wobei die schwere Fraktion (S, V) aus dem zweiten Vortrennschritt (6) oder ein Teil hiervon als der Trenneinsatz (S, V) oder als ein Teil des Trenneinsatzes (S, V) verwendet wird, der dem Ethylenabtrennschritt (7) zugeführt wird.

3. Verfahren (100) nach Anspruch 2, bei dem die leichte Fraktion (Q) aus dem ersten Vortrennschritt (5) insgesamt weniger als 1 Molprozent an Kohlenwasserstoffen mit vier und zumindest fünf Kohlenstoffatomen und im Rest Methan, Ethan, Ethylen und Kohlenwasserstoffe mit drei Kohlenstoffatomen enthält, und bei dem die schwere Fraktion (R) aus dem ersten Vortrennschritt (5) insgesamt weniger als 1 Molprozent Wasserstoff, Methan und Ethylen und im Rest Ethan, Kohlenwasserstoffe mit drei, vier und zumindest fünf Kohlenstoffatomen enthält.

4. Verfahren (100) nach Anspruch 3, bei dem die leichte Fraktion (H) aus dem zweiten Vortrennschritt (6) insgesamt mehr als 99 Molprozent Methan und Wasserstoff und die schwere Fraktion aus dem zweiten Vortrennschritt (6) insgesamt weniger als 1 Molprozent Methan und Wasserstoff und im Rest Ethan, Ethylen und Kohlenwasserstoffe mit drei Kohlenstoffatomen enthält.

5. Verfahren (200) nach Anspruch 2, bei dem die leichte Fraktion (U) aus dem ersten Vortrennschritt (5") insgesamt weniger als 1 Molprozent an Kohlenwasserstoffen mit zumindest fünf Kohlenstoffatomen und im Rest Methan, Ethan, Ethylen und Kohlenwasserstoffe mit drei und vier Kohlenstoffatomen enthält und bei dem die schwere Fraktion (R) aus dem ersten Vortrennschritt (5) insgesamt weniger als 1 Molprozent an Wasserstoff, Methan und Ethylen und im Rest Ethan und Kohlenwasserstoffe mit drei, vier und zumindest fünf Kohlenstoffatomen enthält.

6. Verfahren (100) nach Anspruch 5, bei dem die leichte Fraktion (H) aus dem zweiten Vortrennschritt (6) insgesamt mehr als 99 Molprozent Methan und Wasserstoff und die schwere Fraktion aus dem zweiten Vortrennschritt (6) insgesamt weniger als 1 Molprozent Methan und Wasserstoff und im Rest Ethan, Ethylen und Kohlenwasserstoffe mit drei und vier Kohlenstoffatomen enthält.

7. Verfahren (100, 200) nach einem der vorstehenden Ansprüche 3 bis 6, bei dem die schwere Fraktion (S, V) aus dem ersten Vortrennschritt (5, 5") oder ein Teil hiervon einem weiteren Trennschritt (8) unterworfen wird, in dem eine leichte Fraktion (N) und eine schwere Fraktion (O) gebildet werden, wobei die leichte Fraktion entweder weniger als 1 Molprozent an Kohlenwasserstoffen mit zumindest sechs Kohlenstoffatomen und im Rest Kohlenwasserstoffe mit drei, vier und fünf Kohlenstoffatomen oder weniger als 1 Molprozent an Kohlenwasserstoffen mit zumindest fünf Kohlenstoffatomen und im Rest Kohlenwasserstoffe mit drei und vier Kohlenstoffatomen enthält.

8. Verfahren (300) nach Anspruch 1, bei dem der Trenneinsatz (X), der dem Ethylenabtrennschritt (7) zugeführt wird, unter Verwendung eines einzigen Vortrennschritts (6) gebildet wird, dem das Folgegemisch (F) in unveränderter Zusammensetzung zugeführt wird, und in dem eine leichte Fraktion (H), die insgesamt mehr als 99 Molprozent Methan und Wasserstoff enthält, und eine schwere Fraktion (X), die insgesamt weniger als 1 Molprozent Methan und Wasserstoff und im Rest Ethan, Ethylen und Kohlenwasserstoffe mit drei, vier und zumindest fünf Kohlenstoffatomen enthält, gebildet werden, wobei die schwere Fraktion (X) aus dem einzigen Vortrennschritt (6) oder ein Teil hiervon als der Trenneinsatz (X) oder als ein Teil des Trenneinsatzes (X) verwendet wird, der dem Ethylenabtrennschritt (7) zugeführt wird.

9. Verfahren (300) nach Anspruch 8, bei dem zumindest ein Teil der schweren Fraktion (X) aus dem einzigen Vortrennschritt (6), der nach dem Ethylenabtrennschritt (7) verbleibt, einem weiteren Trennschritt (8') zugeführt wird, in dem eine leichte Fraktion (B) und eine schwere Fraktion (O) gebildet werden, wobei die leichte Fraktion entweder weniger als 1 Molprozent an Kohlenwasserstoffen mit zumindest sechs Kohlenstoffatomen und im Rest Kohlenwasserstoffe mit drei, vier und fünf Kohlenstoffatomen oder weniger als 1 Molprozent an Kohlenwasserstoffen mit zumindest fünf Kohlenstoffatomen und im Rest Kohlenwasserstoffe mit drei und vier Kohlenstoffatomen enthält.

10. Verfahren (100, 200, 300) nach einem der vorstehenden Ansprüche 7 oder 9, bei dem die leichte Fraktion (N, B) aus dem weiteren Trennschritt (8) oder ein Teil hiervon zur Bildung des zweiten Einsatzgases (B) verwendet wird.

11. Verfahren nach einem der vorstehenden Ansprüche 7, 9 oder 10, bei dem die schwere Fraktion (O) aus dem weiteren Trennschritt (8, 8') zusammen mit einer Fraktion (D) aus der Aufbereitung (2), die Kohlenwasserstoffe mit zumindest fünf oder zumindest sechs Kohlenstoffatomen enthält, aus dem Verfahren (100, 200, 300) ausgeführt wird.

## Claims

1. Method (100, 200, 300) for recovering ethylene, wherein a first feed gas (A) and a second feed gas (B) are fed to a reactor (1) and processed therein to obtain a product mixture (C) by steam cracking, wherein the first feed gas (A) has more than 90 percent by weight saturated hydrocarbons and more than 80 percent by weight ethane, and wherein the product mixture (C) or a part thereof is subjected to a preparation (2, 3, 4) to obtain a subsequent mixture (F) containing hydrogen, methane, ethane, ethylene, and hydrocarbons having three, four, and at least five carbon atoms, and the subsequent mixture (F) or a part thereof is subjected to a separation (10), **characterized in that** the separation (10) comprises an ethylene separation step (7) to which at least the ethane, the ethylene, and the hydrocarbons having three carbon atoms from the subsequent mixture (F) or a part thereof in each case are fed to a common separation feed (S, V, X) without being separated, wherein, in which, in the ethylene separation step (7), a light fraction (K), which contains more than 95 mole percent ethylene, and a heavy fraction (T, W, Y), which at least a part of the ethane from the separation feed (S, V, X) and at least 15 percent by weight of the hydrocarbons having three and four carbon atoms from the separation feed (S, V, X), are formed, and wherein the heavy separation product (T, W, Y) from the ethylene separation step (7) or a part thereof is used as a part of or to form the second feed gas (B).

2. Method (100, 200) according to claim 1, wherein the separation feed (S, V) fed to the ethylene separation step (7) is formed using a first pre-separation step (5, 5") and a second pre-separation step (6), wherein the subsequent mixture (F) or part thereof subjected to the separation (10) is fed to the first separation step (5, 5") in unchanged composition, wherein, in the first pre-separation step (5, 5"), a light fraction (Q, U) and a heavy fraction (R) are formed, wherein the light fraction (Q, U) from the first pre-separation step (5, 5") or a part thereof is fed to the second pre-separation step (6), wherein, in the second pre-separation step (6), a light fraction (H) and a heavy fraction (S, V) are formed, and wherein the heavy fraction (S, V) from the second pre-separation step (6) or a part thereof is used as the separation feed (S, V) or as a part of the separation feed (S, V) which is fed to the ethylene separation step (7).

3. Method (100) according to claim 2, wherein the light fraction (Q) from the first pre-separation step (5) contains in total less than 1 mole percent of hydrocarbons having four and at least five carbon atoms and, in the remainder, methane, ethane, ethylene, and hydrocarbons having three carbon atoms, and wherein the heavy fraction (R) from the first pre-separation step (5) contains in total less than 1 mole percent hydrogen, methane, and ethylene and, in the remainder, ethane, hydrocarbons having three, four, and at least five carbon atoms.

4. Method (100) according to claim 3, wherein the light fraction (H) from the second pre-separation step (6) contains in total more than 99 mole percent methane and hydrogen, and the heavy fraction from the second pre-separation step (6) contains in total less than 1 mole percent methane and hydrogen and, in the remainder, ethane, ethylene, and hydrocarbons having three carbon atoms.

5. Method (200) according to claim 2, wherein the light fraction (U) from the first pre-separation step (5") contains in total less than 1 mole percent of hydrocarbons having at least five carbon atoms and, in the remainder, methane, ethane, ethylene, and hydrocarbons having three and four carbon atoms, and wherein the heavy fraction (R) from the first pre-separation step (5) contains in total less than 1 mole percent hydrogen, methane, and ethylene and, in the remainder, ethane and hydrocarbons having three, four, and at least five carbon atoms.

6. Method (100) according to claim 5, wherein the light fraction (H) from the second pre-separation step (6) contains in total more than 99 mole percent methane and hydrogen, and the heavy fraction from the second pre-separation step (6) contains in total less than 1 mole percent methane and hydrogen and, in the remainder, ethane, ethylene, and hydrocarbons having three and four carbon atoms.

7. Method (100, 200) according to one of the preceding claims 3 through 6, wherein the heavy fraction (S, V) from the first pre-separation step (5, 5") or a part thereof is subjected to a further separation step (8) in which a light fraction (N) and a heavy fraction (O) are formed, wherein the light fraction contains either less than 1 mole percent of hydrocarbons having at least six carbon atoms and, in the remainder, hydrocarbons having three, four, and five carbon atoms, or less than 1 mole percent of hydrocarbons having at least five carbon atoms and, in the remainder, hydrocarbons having three and four carbon atoms.

8. Method (300) according to claim 1, wherein the separation feed (X), which is fed to the ethylene separation step (7), is formed using a single pre-separation step (6) to which the subsequent mixture (F) is fed in unchanged composition, and in which a light fraction (H), which contains in total more than 99 mole percent methane and hydrogen, and a heavy fraction (X), which contains in total less than 1 mole percent methane and hydrogen and, in the remainder, ethane, ethylene, and hydrocarbons having three, four, and at least five carbon atoms, are formed, wherein the heavy fraction (X) from the single pre-separation step (6) or part thereof is used as the separation feed (X) or as a part of the separation feed (X) which is fed to the ethylene separation step (7).

9. Method (300) according to claim 8, wherein at least a part of the heavy fraction (X) from the single pre-separation step (6) remaining after the ethylene separation step (7) is fed to a further separation step (8') in which a light fraction (B) and a heavy fraction (O) are formed, wherein the light fraction contains either less than 1 mole percent of hydrocarbons having at least six carbon atoms and, in the remainder, hydrocarbons having three, four, and five carbon atoms, or less than 1 mole percent of hydrocarbons having at least five carbon atoms and, in the remainder, hydrocarbons having three and four carbon atoms.

10. Method (100, 200, 300) according to one of the preceding claims 7 or 9, wherein the light fraction (N, B) from the further separation step (8) or a part thereof is used to form the second feed gas (B).

11. Method according to one of the preceding claims 7, 9, or 10, wherein the heavy fraction (O) from the further separation step (8, 8') together with a fraction (D) from the preparation (2) which contains hydrocarbons having at least five or at least six carbon atoms is realized from the method (100, 200, 300).

## Revendications

1. Procédé (100, 200, 300) de récupération d'éthylène, selon lequel un premier gaz de charge (A) et un second gaz de charge (B) sont amenés à un réacteur (1) et traités dans celui-ci par craquage à la vapeur d'eau avec obtention d'un mélange produit (C), où le premier gaz de charge (A) comporte plus de 90 % en poids d'hydrocarbures saturés et plus de 80 % en poids d'éthane et où le mélange produit (C), ou une partie de celui-ci, avec obtention d'un mélange secondaire (F) contenant de l'hydrogène, du méthane, de l'éthane, de l'éthylène ainsi que des hydrocarbures comprenant trois, quatre et au moins cinq atomes de carbone, est soumis à un traitement (2, 3, 4) et le mélange secondaire (F), ou une partie de celui-ci, est soumis à une séparation (10), **caractérisé en ce que** la séparation (10) comprend une étape de séparation d'éthylène (7), dans laquelle au moins l'éthane, l'éthylène et les hydrocarbures comprenant trois atomes de carbone du mélange secondaire (F), ou à chaque fois une partie de ceux-ci, sont amenés séparés les uns des autres dans une charge de séparation (S, V, X) commune, où, dans l'étape de séparation d'éthylène (7), une fraction légère (K), laquelle contient plus de 95 % en mole d'éthylène, et une fraction lourde (T, W, Y), laquelle contient au moins une partie de l'éthane provenant de la charge de séparation (S, V, X) et au moins 15 % en poids des hydrocarbures comprenant trois et quatre atomes de carbone de la charge de séparation (S, V, X), sont formées, et où le produit de séparation lourd (T, W, Y) de l'étape de séparation d'éthylène (7), ou une partie de celui-ci, est utilisé en tant que partie ou pour la formation du second gaz de charge (B).

2. Procédé (100, 200) selon la revendication 1, selon lequel la charge de séparation (S, V), laquelle est amenée à l'étape de séparation d'éthylène (7), est formée à l'aide d'une première étape de pré-séparation (5, 5") et d'une seconde étape de pré-séparation (6), où, à la première étape de pré-séparation (5, 5"), le mélange secondaire (F) ou sa partie soumise à la séparation (10) est amené(e) dans une composition inchangée, où, dans la première étape de pré-séparation (5, 5"), une fraction légère (Q, U) et une fraction lourde (R) sont formées, où la fraction légère (Q, U) de la première étape de pré-séparation (5, 5"), ou une partie de celle-ci, est amenée à la seconde étape de pré-séparation (6), où, dans la seconde étape de pré-séparation (6), une fraction légère (H) et une fraction lourde (S, V) sont formées et où la fraction lourde (S, V) de la seconde étape de pré-séparation (6), ou une partie de celle-ci, est utilisée en tant que charge de séparation (S, V) ou en tant qu'une partie de la charge de séparation (S, V), laquelle est amenée à l'étape de séparation d'éthylène (7).

3. Procédé (100) selon la revendication 2, selon lequel la fraction légère (Q) de la première étape de pré-séparation (5) contient au total moins de 1 % en mole d'hydrocarbures comprenant quatre et au moins cinq atomes de carbone et dans le reste du méthane, de l'éthane, de l'éthylène et des hydrocarbures comprenant trois atomes de carbone et selon lequel la fraction lourde (R) de la première étape de pré-séparation (5) contient au total moins de 1 % en mole d'hydrogène, de méthane et d'éthylène et dans le reste de l'éthane, des hydrocarbures comprenant trois, quatre et au moins cinq atomes de carbone.

4. Procédé (100) selon la revendication 3, selon lequel la fraction légère (H) de la seconde étape de pré-séparation (6) contient au total plus de 99 % en mole de méthane et d'hydrogène et la fraction lourde de la seconde étape de pré-séparation (6) contient au total moins de 1 % en mole de méthane et d'hydrogène et dans le reste de l'éthane, de l'éthylène et des hydrocarbures comprenant trois atomes de carbone.

5. Procédé (200) selon la revendication 2, selon lequel la fraction légère (U) de la première étape de pré-séparation (5") contient au total moins de 1 % en mole d'hydrocarbures comprenant au moins cinq atomes de carbone et dans le reste du méthane, de l'éthane, de l'éthylène et des hydrocarbures comprenant trois et quatre atomes de carbone et selon lequel la fraction lourde (R) de la première étape de pré-séparation (5) contient au total moins de 1 % en mole d'hydrogène, de méthane et d'éthylène et dans le reste de l'éthane et des hydrocarbures comprenant trois, quatre et au moins cinq atomes de carbone.

6. Procédé (100) selon la revendication 5, selon lequel la fraction légère (H) de la seconde étape de pré-séparation (6) contient au total plus de 99 % en mole de méthane et d'hydrogène et la fraction lourde de la seconde étape de pré-séparation (6) contient au total moins de 1 % en mole de méthane et d'hydrogène et dans le reste de l'éthane, de l'éthylène et des hydrocarbures comprenant trois et quatre atomes de carbone.

7. Procédé (100, 200) selon l'une quelconque des revendications précédentes 3 à 6, selon lequel la fraction lourde (S, V) de la première étape de pré-séparation (5, 5"), ou une partie de celle-ci, est soumise à une autre étape de séparation (8) dans laquelle une fraction légère (N) et une fraction lourde (O) sont formées, où la fraction légère contient soit moins de 1 % en mole d'hydrocarbures comprenant au moins six atomes de carbone et dans le reste des hydrocarbures comprenant trois, quatre et cinq atomes de carbone, soit moins de 1 % en mole d'hydrocarbures comprenant au moins cinq atomes de carbone et dans le reste des hydrocarbures comprenant trois et quatre atomes de carbone.

8. Procédé (300) selon la revendication 1, selon lequel la charge de séparation (X), laquelle est amenée à l'étape de séparation d'éthylène (7), est formée à l'aide d'une seule étape de pré-séparation (6) dans laquelle le mélange secondaire (F) est amené dans la composition inchangée et dans laquelle la fraction légère (H), laquelle contient au total plus de 99 % en mole de méthane et d'hydrogène, et une fraction lourde (X), laquelle contient au total moins de 1 % en mole de méthane et d'hydrogène et dans le reste de l'éthane, de l'éthylène et des hydrocarbures comprenant trois, quatre et au moins cinq atomes de carbone, sont formées, où la fraction lourde (X) de la seule étape de pré-séparation (6), ou une partie de celle-ci, est utilisée en tant que charge de séparation (X) ou en tant que partie de la charge de séparation (X), laquelle est amenée à l'étape de séparation d'éthylène (7).

9. Procédé (300) selon la revendication 8, selon lequel au moins une partie de la fraction lourde (X) de la seule étape de pré-séparation (6), laquelle reste après l'étape de séparation d'éthylène (7), est amenée à une autre étape de séparation (8') dans laquelle une fraction légère (B) et une fraction lourde (O) sont formées, où la fraction légère contient soit moins 1 % en mole d'hydrocarbures comprenant au moins six atomes de carbone et dans le reste des hydrocarbures comprenant trois, quatre et cinq atomes de carbone, soit moins de 1 % en mole d'hydrocarbures comprenant au moins cinq atomes de carbone et dans le reste des hydrocarbures comprenant trois et quatre atomes de carbone.

10. Procédé (100, 200, 300) selon l'une quelconque des revendications précédentes 7 ou 9, selon lequel la fraction légère (N, B) de l'autre étape de séparation (8), ou d'une partie de celle-ci, est utilisée pour former le second gaz de charge (B).

11. Procédé selon l'une quelconque des revendications précédentes 7, 9 ou 10, selon lequel la fraction lourde (O) de l'autre étape de séparation (8, 8'), conjointement avec une fraction (D) du traitement (2), laquelle contient les hydrocarbures comprenant au moins cinq ou au moins six atomes de carbone, sont réalisées selon le procédé (100, 200, 300).
